# FASCICULE DE BREVET EUROPEEN

(11) **EP 2 914 189 B1**
(45) Date de publication et mention de la délivrance du brevet: **09.05.2018**
(21) Numéro de dépôt: 13805451.5
(22) Date de dépôt: 31.10.2013
(51) Int. Cl.: A61B 17/86

(54) **VIS INTRAOSSEUSE POUR FIXER À UN OS UN FRAGMENT OSSEUX OU UN TRANSPLANT**
INTRAOSSALE SCHRAUBE ZUM BEFESTIGEN EINES KNOCHENFRAGMENTS ODER EINES TRANSPLANTATS AN EINEM KNOCHEN
INTRAOSSEOUS SCREW FOR FIXING A BONE FRAGMENT OR A TRANSPLANT TO A BONE

(30) Priorité: 05.11.2012 FR 1260471
(43) Date de publication de la demande: 09.09.2015
(73) Titulaire: De Lavigne Sainte Suzanne, Christophe, 33000 Bordeaux (FR)
(72) Inventeur: De Lavigne Sainte Suzanne, Christophe, 33000 Bordeaux (FR)
(74) Mandataire: Chevalier, Renaud Philippe
(86) Numéro de dépôt international: PCT/FR2013/052616
(87) Numéro de publication internationale: WO 2014/068259

(56) Documents cités:
- WO-A1-96/40020
- WO-A1-2008/092192
- WO-A2-2008/021474
- US-A1- 2004 015 172
- US-A1- 2009 319 043

## Description

La présente invention concerne une vis intraosseuse à implanter dans un os.

La présente invention peut s'appliquer dans tout domaine médical où il faut implanter une vis intraosseuse dans un os. Par ailleurs, la présente invention peut s'appliquer en ostéosynthèse pour fixer un fragment osseux à un os, généralement pour réparer une fracture. La présente invention peut aussi s'appliquer en ligamentoplastie pour fixer un transplant à un os, en passant ce transplant dans un tunnel osseux.

Dans l'art antérieur, pour poser une vis d'ostéosynthèse ou d'interférence, il faut utiliser une mèche ou un foret, afin de préparer un tunnel pour faire pénétrer la tête et l'âme de vis au milieu du pré-trou foré. Certaines vis résorbables sont moulées par injection en acide polylactique (PLA), en un mélange acide polylactique (PLA) et d'hydroxyapatite ou en un mélange acide polylactique (PLA) et de triphosphate de calcium.

Cependant, ces vis en matériaux résorbables moulées par injection ne sont pas assez perméables au milieu liquide pour permettre une résorption rapide, c'est-à-dire dans le temps imparti pour une bonne intégration osseuse.

De plus, on connaît des vis intraosseuses qui présentent un logement pour recevoir des morceaux d'os concassé ou un insert de fixation d'un ligament. Ce type de vis intraosseuses de l'art antérieur a des parois pleines pour la résistance mécanique de la vis intraosseuse. Ces parois pleines sont issues de moulage et sont donc essentiellement imperméables. Une telle vis intraosseuse présente parfois quelques fenêtres localisées dans le but d'alléger la vis intraosseuse. De plus, une vis de l'art antérieur possède une tête de vis pour perforer l'os.

Cependant, une telle vis intraosseuse traumatise l'os, car elle nécessite de découper une portion d'os, ce qui diminue le capital osseux. De plus, comme leurs parois sont pleines, la reconstruction osseuse ne peut pas se produire dans l'orifice où est logée la vis intraosseuse. Donc, une telle vis intraosseuse ne permet pas ou peu de reconstruction de tissu osseux dans le logement.

Le document US2009/0319043 A1 décrit une vis intraosseuse conforme au préambule de la revendication 1. La présente invention vise notamment à résoudre, en tout ou partie, les problèmes mentionnés ci-avant.

A cet effet, l'invention a pour objet une vis intraosseuse, destinée à fixer à un os un fragment osseux pour une ostéosynthèse ou un transplant pour une ligamentoplastie, la vis intraosseuse comprenant :
- au moins un filetage externe qui est formé par une pluralité de filets et qui s'étend suivant une hélice dite externe autour d'un axe longitudinal ;
- un logement qui s'étend à l'intérieur du filetage externe et globalement parallèlement à l'axe longitudinal ;
- des moyens de liaison agencés de façon à relier les filets entre eux ;
les moyens de liaison comprenant des entretoises, chaque entretoise reliant de préférence au moins deux filets consécutifs, les entretoises étant agencées de façon à définir une multitude d'orifices traversants qui débouchent dans le logement,
et le logement délimitant un volume cylindrique creux qui débouche en au moins une extrémité du filetage externe, le volume cylindrique creux étant conformé pour loger, après vissage de la vis intraosseuse dans l'os, une portion d'os cylindrique,
caractérisé en ce que le volume cylindrique creux a un diamètre sensiblement égal au diamètre interne de la portion d'extrémité du filetage externe. Cette portion d'os cylindrique peut le plus souvent provenir de l'os du patient. Ainsi, une telle vis intraosseuse peut être vissée dans un os via le filetage externe. De plus, des ostéoblastes peuvent s'établir dans le logement et à travers les nombreux orifices traversants, permettant ainsi une reconstruction osseuse rapide dans et autour de la vis intraosseuse.

Les moyens de liaison supportent le filetage externe, ce qui confère une résistance mécanique à la vis intraosseuse. Chaque orifice traversant est défini par des entretoises et, le plus souvent, aussi par des filets du filetage externe. Les entretoises permettent de ménager des orifices traversants relativement grands, tout en conférant une grande résistance mécanique au filetage externe, donc à la vis intraosseuse. Typiquement, la multitude d'orifices traversants peut comprendre plusieurs dizaines, voire plusieurs centaines d'orifices traversants délimités par les entretoises.

Dans certaines opérations (ligamentoplastie avec vis intraosseuse dite d'interférence), une carotte osseuse peut être insérée dans le logement. Les orifices traversants permettent au sang de s'écouler vers et hors du logement, de façon à irriguer la carotte osseuse.

Alternativement, dans le cas d'une ostéosynthèse, pour la réparation osseuse, une vis intraosseuse conforme à l'invention peut être vissée dans l'os, après une éventuelle préparation de l'os par une tréphine, ce qui isole une portion d'os dans le logement de la vis intraosseuse. Dans cette alternative, on ne creuse pas un alésage avec perte osseuse totale à l'emplacement occupé par le foret. Au contraire, la tréphine n'enlève pas l'os initial du patient et évide seulement un volume minimal que doit occuper la vis intraosseuse. Après vissage de la vis intraosseuse, les orifices traversants permettent au sang de s'écouler vers et hors du logement, de façon à irriguer cette portion d'os qui a été isolée de l'os du patient mais pas retirée à l'os du patient.

De manière connue en soi, la vis intraosseuse peut être introduite à l'aide un tournevis. Le culot de la vis intraosseuse peut être réalisé suivant tout modèle existant pour tout type de tournevis, en particulier dans le cas d'une vis intraosseuse pour ostéosynthèse, qui ne nécessite pas de réintroduire de carotte osseuse par l'arrière de la vis intraosseuse.

Selon une variante de l'invention, les filets forment un filetage externe continu. En d'autres termes, ces filets sont intègres, donc pas sectionnés. Ainsi, un tel filetage externe présente une résistance mécanique élevée.

Selon une variante de l'invention, la vis intraosseuse comprend au moins deux filetages externes qui s'enroulent suivant deux hélices parallèles et de pas différents autour de l'axe longitudinal. Ainsi, de tels filetages externes peuvent reprendre des efforts mécaniques importants de type compression d'une zone fracturaire.

Selon une variante de l'invention, les moyens de liaison forment un noyau cylindrique coaxial à l'axe longitudinal, ledit au moins un filetage externe s'étendant autour du noyau. Ainsi, la vis intraosseuse peut être fabriquée en solidarisant, par exemple par collage, le noyau au filetage externe.

Selon une variante de l'invention, ledit au moins un filetage externe étant composé d'au moins un matériau biocompatible et biorésorbable. Ainsi, la vis intraosseuse peut être résorbée partiellement ou complètement.

Selon un mode de réalisation de l'invention, les dimensions transversales du logement sont supérieures à 90% de la dimension minimale de la partie interne du filetage externe.

Ainsi, le logement peut loger une portion d'os cylindrique de diamètre relativement grand, ce qui conserve le capital osseux et donne une résistance mécanique élevée à cette portion d'os.

Selon un mode de réalisation de l'invention, le volume cylindrique creux débouche aux deux extrémités du filetage externe, de sorte que, après vissage de la vis intraosseuse dans l'os, le filetage externe peut loger une portion d'os restant solidaire de l'os.

En d'autres termes, la vis intraosseuse est dépourvue de tête de vis, au moins à une extrémité de la vis intraosseuse. Ainsi, une telle vis intraosseuse (vis d'interférence pour ostéosynthèse) peut être serrée dans l'os sans enlèvement préalable d'une carotte osseuse, ce qui minimise la perte osseuse du patient.

Selon l'invention, le volume cylindrique creux du logement a un diamètre sensiblement égal au diamètre interne de la portion d'extrémité du filetage externe.

Ainsi, le logement peut loger une portion d'os cylindrique de grand diamètre, ce qui conserve le capital osseux.

Selon un mode de réalisation de l'invention, les entretoises se croisent au moins deux à deux.

En d'autres termes, deux entretoises voisines forment une intersection. Ainsi, de tels croisements ou intersections augmentent la résistance mécanique de la vis intraosseuse.

Selon un mode de réalisation de l'invention, les entretoises comprennent :
- des entretoises dites inverses qui s'étendent globalement suivant des hélices dites inverses qui sont orientées inversement à l'hélice externe dudit au moins un filetage externe ; et
- des entretoises dites directes qui s'étendent globalement suivant des hélices dites directes qui sont orientées comme l'hélice externe dudit au moins un filetage externe.

En d'autres termes, lorsque le filetage externe suit une hélice externe qui est dextre (pas à droite), les entretoises inverses suivent des hélices inverses qui sont sénestres (pas à gauche). Lorsque le filetage externe suit une hélice externe qui est dextre (pas à droite), les entretoises directes suivent des hélices directes qui sont dextres (pas à droite).

Ainsi, la présence d'entretoises directes et d'entretoises inverses réalise un maillage croisé comme un grillage, ce qui confère à la vis intraosseuse une résistance mécanique à tous les efforts qu'elle doit supporter. En particulier, les entretoises inverses résistent à des efforts, notamment en torsion autour de l'axe longitudinal, auxquels le filetage externe résiste peu.

Selon une variante de l'invention, la vis intraosseuse présente des noeuds entre deux filets consécutifs, chaque noeud étant formé par l'intersection d'au moins deux entretoises.

Ainsi, de tels noeuds ou croisillons augmentent la résistance mécanique de la vis intraosseuse.

Selon un mode de réalisation de l'invention, ledit au moins un filetage externe présente plusieurs canaux dits capillaires qui traversent ledit au moins un filetage externe de façon à déboucher dans le logement.

Ainsi, de tels canaux capillaires contribuent largement à la circulation du sang vers et hors du logement, donc à la reconstruction osseuse dans le logement et à la résorption de la vis intraosseuse.

Selon un mode de réalisation de l'invention, des entretoises, de préférence la majorité des entretoises, présentent des canaux capillaires traversants de façon à déboucher dans le logement.

Ainsi, de tels canaux capillaires contribuent largement à la circulation du sang vers et hors du logement, donc à la reconstruction osseuse dans le logement et à la résorption de la vis intraosseuse.

Dans la présente demande, le terme « canal capillaire » désigne un canal qui permet l'écoulement de sang par capillarité. Les dimensions d'un canal capillaire sont telles qu'il se produit, entre ses extrémités, un effet de pompage. Cet effet de pompage constaté est causé par effet capillaire, dus aux capillaires résultant d'un maillage de fibres, par effet venturi, par échanges gazeux entre l'os du patient et la portion cylindrique placée dans le logement cylindrique creux.

Selon une variante de l'invention, les canaux capillaires sont répartis régulièrement, de préférence uniformément, sur le filetage externe. Ainsi, cette répartition régulière des canaux capillaires favorise particulièrement la reconstruction osseuse.

Selon un mode de réalisation de l'invention, des canaux capillaires et/ou des orifices traversants ont des formes convergentes depuis une surface externe du filetage externe vers le logement, et dans laquelle des canaux capillaires et/ou des orifices traversants ont des formes divergentes depuis une surface externe du filetage externe vers le logement.

Ainsi, de telles formes convergentes et divergentes favorisent l'écoulement du sang par capillarité à travers ces canaux capillaires et ces orifices traversants. Les canaux convergents favorisent l'entrée du liquide dans le logement, tandis que les canaux divergents favorisent la sortie de liquide hors du logement.

Selon une variante de l'invention, des canaux capillaires, de préférence tous les canaux capillaires, ont des dimensions transversales inférieures à 1,5 mm, de préférence inférieures à 1 mm.

Ainsi, de telles dimensions transversales permettent une bonne circulation du sang par capillarité à travers les canaux capillaires et/ou les orifices traversants.

Selon un mode de réalisation de l'invention, ledit au moins un filetage externe et les moyens de liaison sont respectivement composés de fibres agglomérées.

Ainsi, en plus des canaux capillaires et des orifices traversants qui sont relativement larges et où le sang s'écoule vite, la vis intraosseuse présente des capillaires secondaires, qui s'étendent le long des fibres agglomérées et qui sont plus étroits. Le sang s'écoule plus lentement à travers ces capillaires secondaires qu'à travers les canaux capillaires et les orifices traversants, mais le sang peut y parcourir une distance plus grande. De plus, de telles fibres agglomérées rendent la vis intraosseuse à la fois mécaniquement résistante et relativement légère.

Une telle vis intraosseuse peut être fabriquée en fibres résorbables par filature centrifuge, de préférence dite « rotary jet spinning » ou par filature électrostatique dite « *electrospinning* », dans laquelle un fluide est projeté suivant un mouvement rotatoire de façon à agglomérer des fibres qui sont orientées et positionnées au moyen d'un champ électrostatique.

Selon un mode de réalisation de l'invention, les fibres agglomérées comprennent des fibres composées de polymère d'acide lactique (PLA), et le cas échéant des fibres biorésorbables qui sont de préférence composées de matériaux sélectionnés dans le groupe constitué du collagène, de l'hydroxyapatite et de caprolactone.

Ainsi, de telles fibres sont biocompatibles et elles peuvent être agglomérées par divers procédés, comme par exemple la filature électrostatique (en anglais « *electrospinning* ») ou la filature centrifuge (en anglais « rotary jet spinning »), ou par rotomoulage, ou encore tissées puis enroulées autour d'un axe.

Selon un mode de réalisation de l'invention, les fibres agglomérées comprennent en outre des fibres de strontium.

Ainsi, les fibres de strontium rendent la vis intraosseuse radioopaque, ce qui facilite l'imagerie médicale par radiographie X ou avec un amplificateur de brillance.

Selon une variante de l'invention, tout ou partie des fibres agglomérées peuvent être recouvertes de particules sphéroidales en matériau céramique-verre bioactif, les particules sphéroidales ayant de préférence une dimension inférieure à 2 µm, de préférence inférieure à 1 µm.

Ainsi, de telles particules sphéroidales facilitent l'adhésion osseuse. Un tel matériau céramique-verre bioactif est commercialisé par exemple sous le nom « bioglass ».

Selon un mode de réalisation de l'invention, la vis intraosseuse comprend en outre une tête de vis agencée de sorte que la vis intraosseuse peut être serrée dans un alésage après enlèvement préalable d'une portion d'os globalement cylindrique.

Ainsi, une telle vis intraosseuse remplit la fonction de vis d'interférence et permettant de fixer un transplant à un os, par exemple pour une opération de ligamentoplastie. La portion d'os globalement cylindrique peut être ensuite réinsérée dans le logement.

Selon une variante de l'invention, chaque filet a une épaisseur comprise entre 0,5 mm et 1,5 mm, de préférence entre 0,8 mm et 1,2 mm.

Ainsi, un tel filetage externe peut être adapté à diverses opérations. Les dimensions du filetage externe permettent d'implanter rapidement et durablement la vis intraosseuse et de minimiser la taille du trou à forer dans l'os au moyen d'une tréphine, donc la perte osseuse dans le cas d'une ostéosynthèse.

Selon une variante de l'invention, chaque filet présente un sommet de filet ou une tête de filet dont le profil est arrondi. Ainsi, un tel profil arrondi évite que la tête de filet ne cisaille localement un tissu tendineux ou tissu artificiel d'interférence.

Selon une variante de l'invention, chaque filet présente un profil globalement en forme de « Y », le pied du « Y » formant la tête du filet respectif.

Ainsi, un tel profil en « Y » permet de minimiser l'épaisseur du filetage externe à résorber et de maximiser la taille du logement dans lequel le tissu va croître.

Selon un mode de réalisation de l'invention, la vis intraosseuse comprend en outre un filetage dit interne qui s'étend sur les bords du logement.

Ainsi, un tel filetage interne permet de visser un insert servant à fixer un transplant à la vis intraosseuse, donc à un os, pour une opération de ligamentoplastie.

Selon une première variante de ce mode de réalisation de l'invention, la vis intraosseuse comprend un filetage interne s'étendant sensiblement sur toute la longueur de la vis intraosseuse, de façon à augmenter sa stabilité sur l'os (ostéoporose). Selon une deuxième variante de ce mode de réalisation de l'invention, la vis intraosseuse comprend un filetage interne s'étendant seulement sur une partie postérieure de la vis intraosseuse, de façon à fixer un transplant à la vis intraosseuse.

Avantageusement, le filetage interne s'étend seulement sur une portion postérieure de la vis intraosseuse.

Selon une variante de l'invention, la vis intraosseuse comprend en outre un oeillet ou équivalent solidarisé à une extrémité postérieure de la vis intraosseuse. Ainsi, un tel oeillet permet de former une ancre creuse, sans tête, l'oeillet servant à passer un double fil d'ancrage inséré à l'arrière de la vis, dans le but de fixer des attaches ligamentaires.

Selon une variante de l'invention, le filetage externe a une longueur comprise entre 1 mm et 10 mm, de préférence entre 1 mm et 5 mm.

Selon un mode de réalisation de l'invention, les entretoises sont formées par des tiges, de préférence à section globalement circulaire.

Selon un mode de réalisation de l'invention, chaque entretoise s'étend entre deux filets consécutifs. En d'autres termes, chaque entretoise est délimitée par deux filets consécutifs. Autrement dit, chaque entretoise présente une première extrémité disposée sur un filet et une deuxième extrémité disposée sur le filet consécutif.

Ainsi, les entretoises et le filetage externe sont des pièces distinctes, ce qui simplifie la fabrication de la vis intraosseuse, en particulier de petites dimensions, et ce qui confère au filetage externe une résistance mécanique élevée.

Selon un mode de réalisation de l'invention, les entretoises sont configurées de sorte que la superficie cumulée des orifices traversants représente au moins 50%, de préférence au moins 70%, de la surface délimitée entre les filets.

En d'autres termes, la majeure partie de cette surface est ouverte. Cette surface a une forme globalement hélicoïdale. Ainsi, une telle vis intraossseuse favorise grandement la croissance osseuse à travers la vis intraossseuse.

Selon un mode de réalisation de l'invention, la vis intraosseuse comprend au moins 5 entretoises, de préférence au moins 8 entretoises, entre deux filets consécutifs.

Par ailleurs, la présente description présente un procédé de fabrication, pour fabriquer une vis intraosseuse selon l'invention, le procédé de fabrication comprenant des étapes de projection de fibres dans un moule dont l'empreinte définit la vis intraosseuse, la projection étant de préférence réalisée par filature centrifuge dite « *rotary jet spinning* » ou par filature électrostatique sous la commande d'un calculateur.

Ainsi, un tel procédé de fabrication permet de fabriquer des vis intraosseuses de petite taille à un coût raisonnable. Alternativement, la projection des fibres peut être réalisée par filature centrifuge (en anglais « rotary jet spinning ») ou par filature rotomoulée conventionnelle d'un tissé autour d'un axe.

Alternativement à ce procédé de fabrication, il est possible de fabriquer une vis intraosseuse conforme à l'invention en réalisant un procédé génératif, tel que du frittage sélectif au laser.

Les modes de réalisation et les variantes mentionnés ci-avant peuvent être pris isolément ou selon toute combinaison techniquement admissible.

La présente invention sera bien comprise et ses avantages ressortiront aussi à la lumière de la description qui va suivre, donnée uniquement à titre d'exemple non limitatif et faite en référence aux dessins annexés, dans lesquels :
- la figure 1 est une vue en perspective d'une vis intraosseuse sans tête conforme à un premier mode de réalisation de l'invention ;
- la figure 2 est une vue en perspective, suivant un angle différent de la figure 1, de la vis intraosseuse de la figure 1 ;
- la figure 3 est une vue en perspective de face de la vis intraosseuse de la figure 1 ;
- la figure 4 est une vue à plus grande échelle d'une partie de la vis intraosseuse de la figure 1 ;
- la figure 5 est une coupe suivant un plan V à la figure 4 ;
- la figure 6 est une vue similaire à la figure 2 d'une vis intraosseuse sans tête conforme à un deuxième mode de réalisation de l'invention ;
- la figure 7 est une vue en perspective d'une vis intraosseuse avec tête conforme à un troisième mode de réalisation de l'invention ;
- la figure 8 est une vue en perspective, suivant un angle différent de la figure 7, de la vis intraosseuse de la figure 7 ;
- la figure 9 est une vue schématique d'un canal capillaire formé dans une vis intraosseuse conforme à l'invention ; et
- la figure 10 est une vue microscopique d'une partie d'une une vis intraosseuse conforme à l'invention.

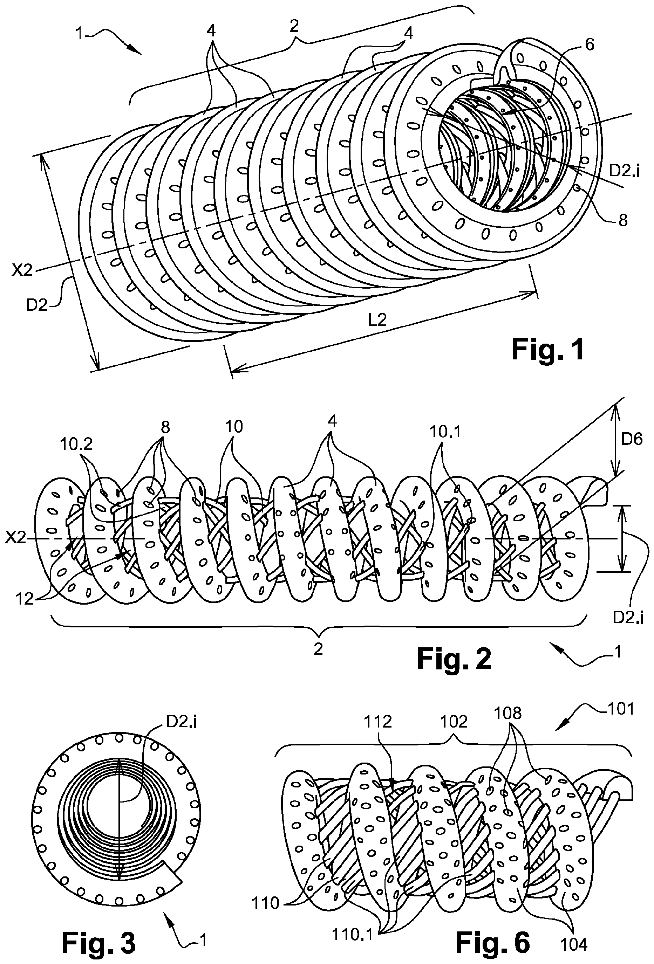

Les figures 1, 2, 3, 4 et 5 illustrent une vis intraosseuse 1 sans tête de vis conforme à un premier mode de réalisation de l'invention. La vis intraosseuse 1 est destinée à fixer à un os un transplant pour une ligamentoplastie.

La vis intraosseuse 1 comprend un filetage externe 2 qui est formé par une pluralité de filets 4. Le filetage externe 2 s'étend suivant une hélice dite externe autour d'un axe longitudinal X2. Dans l'exemple des figures 1 et 2, les filets 4 forment un filetage externe 2 qui est continu.

Chaque filet 4 a ici une épaisseur d'environ 1 mm. Le filetage externe 2 a ici une longueur L2 d'environ 4 mm et un diamètre externe D2, mesuré sur le sommet du filet 4, d'environ 1 mm. Bien entendu, les dimensions, longueur et diamètre externe, d'une vis intraosseuse conforme à l'invention peuvent être adaptées à l'application visée.

De plus, la vis intraosseuse 1 comprend un logement 6 dont une partie est visible à la figure 1. Le logement 6 s'étend à l'intérieur du filetage externe 2 et globalement parallèlement à l'axe longitudinal X2.

Le logement 6 délimite un volume cylindrique creux qui débouche sur les deux extrémités du filetage externe 2 (vis intraosseuse 1 sans tête). Le volume cylindrique creux est conformé de sorte que, après vissage de la vis intraosseuse 1 dans l'os, le volume cylindrique creux, donc le filetage externe 2, peut loger une portion d'os, non représentée, qui reste solidaire de l'os et qui est sensiblement cylindrique. Cette portion d'os peut provenir de l'os du patient.

Ainsi, le volume cylindrique creux du logement 6 est conformé pour loger, après vissage de la vis intraosseuse 1 dans l'os, une portion d'os cylindrique. Les dimensions transversales du logement 6 sont supérieures à 90% de la dimension minimale de la partie interne du filetage externe 2. Ici, le volume cylindrique creux du logement 6 a un diamètre D6 sensiblement égal au diamètre interne D2.i de la portion d'extrémité du filetage externe 2.

Le filetage externe 2 présente plusieurs canaux dits capillaires 8 qui traversent le filetage externe 2 de façon à déboucher dans le logement 6. Dans l'exemple des figures 1 à 5, les canaux capillaires 8 sont répartis régulièrement sur le filetage externe 2. Les canaux capillaires 8 sont ici répartis sensiblement uniformément sur le filetage externe 2.

La vis intraosseuse 1 comprend en outre des entretoises 10. Comme le montre la figure 2, la vis intraosseuse 1 comprend environ 10 entretoises 10 entre deux filets consécutifs 4. Chaque entretoise 10 a ici globalement la forme d'une tige à section globalement circulaire.

Chaque entretoise 10 relie deux filets consécutifs 4. Chaque entretoise 10 s'étend ici entre deux filets consécutifs 4. En d'autres termes, chaque entretoise 10 est délimitée par deux filets consécutifs 4. Les entretoises 10 forment ici des moyens de liaison agencés de façon à relier les filets 4 entre eux. Chaque entretoise 10 relie deux filets consécutifs 4 entre eux.

Les entretoises 10, qui composent les moyens de liaison, sont agencées de façon à définir entre elles une multitude d'orifices traversants 12 qui débouchent dans le logement 6. Les orifices traversants 12 sont ici définis par des interstices s'étendant entre des entretoises 10. Chaque orifice traversant 12 est défini par des entretoises 10 et aussi par des filets 4 du filetage externe 2. De plus, des entretoises 10 peuvent aussi présenter des canaux capillaires traversants de façon à déboucher dans le logement 6.

Dans l'exemple des figures 1 et 2, les entretoises 10 sont configurées de sorte que la superficie cumulée des orifices traversants 12 représente environ 90% de la surface délimitée entre les filets 4.

Les entretoises 10 comprennent des entretoises dites inverses 10.1 qui s'étendent globalement suivant des hélices dites inverses qui sont orientées inversement à l'hélice externe du filetage externe 2. Le filetage externe 2 suit une hélice externe qui est dextre (pas à droite). Les hélices inverses ont donc un pas à gauche.

Les entretoises 10 comprennent en outre des entretoises dites directes 10.2 qui s'étendent globalement suivant des hélices dites directes qui sont orientées comme l'hélice externe du filetage externe 2. Les hélices directes ont donc un pas à droite.

De plus, la vis intraosseuse 1 présente des noeuds 14 entre deux filets consécutifs 4. Chaque noeud 14 est ici formé par l'intersection de deux entretoises 10, en général une entretoise inverse 10.1 et une entretoise directe 10.2. Au moins deux entretoises 10.1 et 10.2 voisines se croisent en un noeud 14.

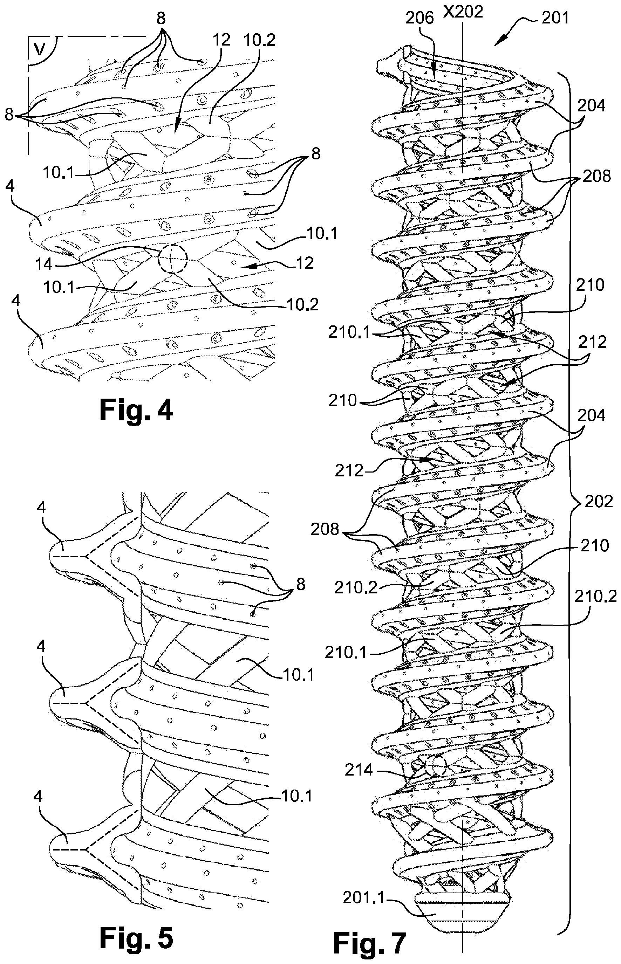

Par ailleurs, chaque filet 4 présente un profil globalement en forme de « Y », le pied du « Y » formant la tête du filet respectif. Chaque filet présente une tête de filet dont le profil est arrondi. Le profil en « Y » des filets 4 est matérialisé en traits pointillés à la figure 5.

Le filetage externe 2 et les entretoises 10, qui forment des moyens de liaison, sont respectivement composés de fibres agglomérées F, visibles à la figure 11. Ces fibres agglomérées comprennent ici des fibres composées de polymère d'acide lactique (PLA), des fibres de collagène et des fibres d'hydroxyapatite.

Les fibres agglomérées comprennent en outre des fibres de strontium, qui rendent la vis intraosseuse 1 radioopaque. De plus, certaines des fibres agglomérées sont recouvertes de particules sphéroïdales en matériau céramique-verre bioactif, les particules sphéroïdales ayant de préférence une dimension inférieure à 2 µm, de préférence inférieure à 1 µm.

Ainsi, de telles sphères facilitent l'adhésion osseuse. Un tel matériau céramique-verre bioactif est commercialisé par exemple sous le nom « bioglass » ou la référence BaG 13.93. Les particules sphéroïdales peuvent être recouvertes de poly-L, DL acide lactique (par exemple connu sous la référence SR-PLA70).

Comme les canaux capillaires 8 et les orifices traversants 14 sont relativement larges, le sang s'y écoulera vite. Lorsqu'elle composée de fibres agglomérées, la vis intraosseuse 1 présente des capillaires secondaires non représentés, qui s'étendent le long des fibres agglomérées et qui sont plus étroits. Le sang s'écoule plus lentement à travers ces capillaires secondaires qu'à travers les canaux capillaires 8 et les orifices traversants 14. Cependant, le sang peut parcourir, par capillarité, une distance plus grande à travers ces capillaires secondaires qu'à travers les canaux capillaires 8 et les orifices traversants 14.

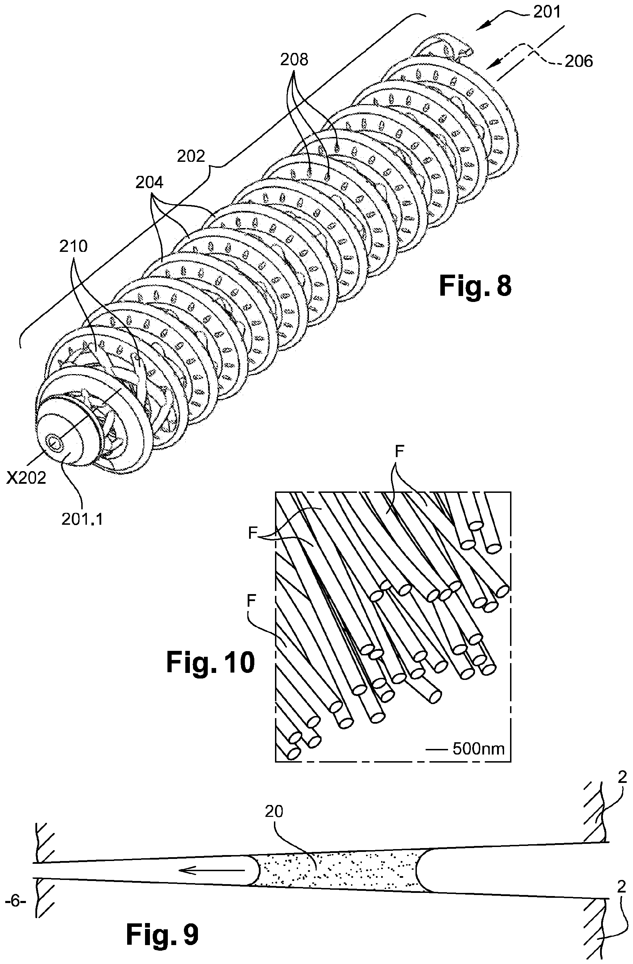

Certains des canaux capillaires 8 ont des formes convergentes depuis une surface externe du filetage externe 2 vers le logement 6. Comme le montre schématiquement la figure 9, un canal capillaire 8 converge de l'amont, situé du côté du filetage externe 2, vers l'aval, situé du côté du logement 6, ce qui permet l'écoulement par capillarité du sang. Inversement, certains des canaux capillaires 8 ont des formes divergentes depuis une surface externe du filetage externe 2 vers le logement 6.

Un décalage régulier des fibres agglomérées permet de former des canaux capillaires 8 de sens aller, vers le logement 6, et des canaux capillaires 8 de sens retour, hors du logement 6. Lorsque le sang peut aller et venir vers et hors du logement 6, il circule partout autour et dans la vis intraosseuse 1, ce qui facilite l'établissement des ostéoblastes et accélère la reconstruction osseuse.

Comme les canaux capillaires 8 et les orifices traversants 14 sont relativement larges, le sang s'y écoulera vite. Lorsqu'elle composée de fibres agglomérées, la vis intraosseuse 1 présente des capillaires secondaires non représentés, qui s'étendent le long des fibres agglomérées et qui sont plus étroits. Le sang s'écoule plus lentement à travers ces capillaires secondaires qu'à travers les canaux capillaires 8 et les orifices traversants 14. Cependant, le sang peut parcourir, par capillarité, une distance plus grande à travers ces capillaires secondaires qu'à travers les canaux capillaires 8 et les orifices traversants 14.

Certains des canaux capillaires 8 ont des formes convergentes depuis une surface externe du filetage externe 2 vers le logement 6. Comme le montre schématiquement la figure 9, un canal capillaire 8 converge de l'amont, situé du côté du filetage externe 2, vers l'aval, situé du côté du logement 6, ce qui permet l'écoulement par capillarité du sang 20. Inversement, certains des canaux capillaires 8 ont des formes divergentes depuis une surface externe du filetage externe 2 vers le logement 6.

Un décalage régulier des fibres agglomérées permet de former des canaux capillaires 8 de sens aller, vers le logement 6, et des canaux capillaires 8 de sens retour, hors du logement 6. Lorsque le sang peut aller et venir vers et hors du logement 6, il circule partout autour et dans la vis intraosseuse 1, ce qui facilite l'établissement des ostéoblastes et accélère la reconstruction osseuse.

La figure 6 illustre une vis intraosseuse 101 conforme à un deuxième mode de réalisation de l'invention. Dans la mesure où la vis intraosseuse 101 est similaire à la vis intraosseuse 1, la description de la vis intraosseuse 1 donnée ci-avant en relation avec les figures 1 à 5 peut être transposée à la vis intraosseuse 101, à l'exception des différences notables énoncées ci-après.

Un composant de la vis intraosseuse 101 identique ou correspondant, par sa structure ou par sa fonction, à un composant de la vis intraosseuse 1 porte la même référence numérique augmentée de 100. On définit ainsi un filetage externe 102, des filets 104, des canaux capillaires 108, des moyens de liaison formés par des entretoises 110 et une multitude d'orifices traversants 112.

La vis intraosseuse 101 diffère de la vis intraosseuse 1, notamment car la vis intraosseuse 101 ne comprend que des entretoises inverses 110.1, alors que la vis intraosseuse 101 comprend aussi des entretoises directes 110.2. Donc les entretoises 110.1 ne se croisent pas ; elles ne forment pas d'intersection entre elles. Les orifices traversants 114 ont donc des formes semblables entre eux, et globalement en parallélogramme incurvé. Ainsi, une telle vis intraosseuse 101 est relativement simple à fabriquer, car elle n'a que des entretoises inverses 110.1. Le nombre et la forme des entretoises 110 sont sélectionnés en fonction de la résistance mécanique souhaitée, donc en fonction notamment de la nature de l'os, par exemple os spongieux ou os cortical. Comme les entretoises 10, des entretoises 110 peuvent présenter des canaux capillaires traversants de façon à déboucher dans le logement 106.

Les figures 7 et 8 illustrent une vis intraosseuse 201 conforme à un troisième mode de réalisation de l'invention. La vis intraosseuse 201 est destinée à fixer à un os un fragment osseux pour une ostéosynthèse en vue d'une réparation osseuse.

La vis intraosseuse 201 comprend un filetage externe 202 qui est formé par une pluralité de filets 204. Le filetage externe 202 s'étend suivant une hélice dite externe autour d'un axe longitudinal X202.

Contrairement à la a vis intraosseuse 1, la vis intraosseuse 201 comprend en outre une tête de vis 201.1. La tête de vis 201.1 est agencée de sorte que la vis intraosseuse 201 peut être serrée dans un alésage autour d'une portion d'os globalement cylindrique, après un éventuel tréphinage de l'os.

Le filetage externe 202 est composé d'au moins un matériau biocompatible. Chaque filet 204 a ici une épaisseur d'environ 1 mm. Le filetage externe 2 a ici une longueur d'environ 4 mm et un diamètre externe, mesuré sur la tête de filet d'environ 1 mm.

De plus, la vis intraosseuse 201 comprend un logement 206 dont une partie est visible à la figure 7. Le logement 206 s'étend à l'intérieur du filetage externe 202 et globalement parallèlement à l'axe longitudinal X202. Le logement 206 délimite un volume cylindrique creux. Le volume cylindrique creux du logement 206 est conformé pour loger, après vissage de la vis intraosseuse 201 dans l'os, une portion d'os cylindrique.

Le filetage externe 202 présente plusieurs canaux dits capillaires 208 qui traversent le filetage externe 202 de façon à déboucher dans le logement 206. Dans l'exemple des figures 7 et 8, les canaux capillaires 208 sont répartis régulièrement sur le filetage externe 202. Les canaux capillaires 208 sont ici répartis sensiblement uniformément sur le filetage externe 202.

La vis intraosseuse 201 comprend en outre des entretoises 210, comme le montrent les figures 7 et 8. Chaque entretoise 210 relie deux filets consécutifs 204. Les entretoises 210 forment ici des moyens de liaison agencés de façon à relier les filets 204 entre eux.

Les entretoises 210, qui composent ces moyens de liaison sont agencées de façon à définir entre elles une multitude d'orifices traversants 212 qui débouchent dans le logement 206. Les orifices traversants 212 sont ici définis par des interstices s'étendant entre des entretoises 210. Dans l'exemple des figures 7 et 8, les entretoises 210 sont configurées de sorte que la superficie cumulée des orifices traversants 212 représente environ 60% de la surface délimitée entre les filets 4.

Dans l'exemple des figures 7 et 8, les entretoises 210 comprennent des entretoises dites inverses 210.1 qui s'étendent globalement suivant des hélices dites inverses qui sont orientées inversement à l'hélice externe du filetage externe 202. Dans l'exemple des figures 7 et 8, le filetage externe 202 suit une hélice externe qui est dextre (pas à droite). Les hélices inverses ont donc un pas à gauche.

Les entretoises 210 comprennent en outre des entretoises dites directes 210.2 qui s'étendent globalement suivant des hélices dites directes qui sont orientées comme l'hélice externe du filetage externe 202. Les hélices directes ont donc un pas à droite.

De plus, la vis intraosseuse 201 présente des noeuds 214 entre deux filets consécutifs 204. Chaque noeud 214 est ici formé par l'intersection de deux entretoises 210, en général une entretoise inverse 210.1 et une entretoise directe 210.2. Au moins deux entretoises 210.1 et 210.2 voisines se croisent en un noeud 214.

Le filetage externe 202 et les entretoises 210, qui forment des moyens de liaison, sont respectivement composés de fibres agglomérées F, visibles à la figure 10. Ces fibres agglomérées comprennent ici des fibres composées de polymère d'acide lactique (PLA), des fibres de collagène, des fibres d'hydroxyapatite et, éventuellement, d'autres fibres résorbables.

Les fibres agglomérées comprennent en outre des fibres de strontium, qui rendent la vis intraosseuse 201 radioopaque. De plus, certaines des fibres agglomérées sont recouvertes de particules sphéroïdales en matériau céramique-verre bioactif, les particules sphéroïdales ayant de préférence une dimension inférieure à 2 µm, de préférence inférieure à 1 µm.

Ainsi, de telles sphères facilitent l'adhésion osseuse. Un tel matériau céramique-verre bioactif est commercialisé par exemple sous le nom « bioglass » ou la référence BaG 13.93. Les particules sphéroïdales peuvent être recouvertes de poly-L, DL acide lactique (par exemple connu sous la référence SR-PLA70).

Comme les canaux capillaires 208 et les orifices traversants 214 sont relativement larges, le sang s'y écoulera vite. Lorsqu'elle composée de fibres agglomérées, la vis intraosseuse 201 présente des capillaires secondaires non représentés, qui s'étendent le long des fibres agglomérées et qui sont plus étroits. Le sang s'écoule plus lentement à travers ces capillaires secondaires qu'à travers les canaux capillaires 208 et les orifices traversants 214. Cependant, le sang peut parcourir, par capillarité, une distance plus grande à travers ces capillaires secondaires qu'à travers les canaux capillaires 208 et les orifices traversants 214.

Pour fabriquer une vis intraosseuse 1, 101 ou 201 conforme à l'invention, un procédé de fabrication comprend des étapes de projection des fibres dans un moule dont l'empreinte définit la vis intraosseuse. Cette projection est réalisée par filature électrostatique (en anglais *« electrospinning* ») ou par rotary jet spinning, sous la commande d'un calculateur. Un fluide est projeté suivant un mouvement rotatoire de façon à agglomérer des fibres qui sont orientées et positionnées au moyen d'un champ électrostatique. Alternativement, La vis intraosseuse 1, 101 ou 201 peut être fabriquée le long d'un axe par fibres projetées ou rotomoulées.

## Revendications

1. Vis intraosseuse (1 ; 101 ; 201), destinée à fixer à un os un fragment osseux pour une ostéosynthèse ou un transplant pour une ligamentoplastie, la vis intraosseuse (1 ; 101 ; 201) comprenant :
- au moins un filetage externe (2 ; 102 ; 202) qui est formé par une pluralité de filets (4 ; 104 ; 204) et qui s'étend suivant une hélice dite externe autour d'un axe longitudinal (X2) ;
- un logement (6) qui s'étend à l'intérieur du filetage externe (2 ; 102 ; 202) et globalement parallèlement à l'axe longitudinal (X2) ; et
- des moyens de liaison agencés de façon à relier les filets (4 ; 104 ; 204) entre eux ; les moyens de liaison comprenant des entretoises (10; 110; 210), chaque entretoise (10; 110; 210) reliant de préférence au moins deux filets consécutifs (4 ; 104 ; 204), les entretoises (10 ; 110 ; 210) étant agencées de façon à définir une multitude d'orifices traversants (12; 112; 212) qui débouchent dans le logement (6),
le logement (6) délimitant un volume cylindrique creux qui débouche en au moins une extrémité du filetage externe (2; 102) et étant conformé pour loger, après vissage de la vis intraosseuse (1 ; 101 ; 201) dans l'os, une portion d'os cylindrique,
la vis intraosseuse étant **caractérisée en ce que** le volume cylindrique creux a un diamètre (D6) sensiblement égal au diamètre interne (D2) de la portion d'extrémité du filetage externe.

2. Vis intraosseuse (1 ; 101 ; 201) selon la revendication 1, dans laquelle les dimensions transversales du logement (6 ; 106 ; 206) sont supérieures à 90% de la dimension minimale de la partie interne du filetage externe (2 ; 102 ; 202).

3. Vis intraosseuse (1 ; 101) selon l'une des revendications précédentes, dans laquelle le volume cylindrique creux débouche aux deux extrémités du filetage externe (2 ; 102), de sorte que, après vissage de la vis intraosseuse (1 ; 101) dans l'os, le filetage externe (2 ; 102) peut loger une portion d'os restant solidaire de l'os.

4. Vis intraosseuse (1 ; 101 ; 201) selon l'une des revendications précédentes, dans laquelle les entretoises (10 ; 110 ; 210) se croisent au moins deux à deux.

5. Vis intraosseuse (1 ; 201) selon la revendication 4, dans laquelle les entretoises (10 ; 210) comprennent :
- des entretoises dites inverses (10.1 ; 210.1) qui s'étendent globalement suivant des hélices dites inverses qui sont orientées inversement à l'hélice externe dudit au moins un filetage externe (2 ; 202) ; et
- des entretoises dites directes (10.2 ; 210.2) qui s'étendent globalement suivant des hélices dites directes qui sont orientées comme l'hélice externe dudit au moins un filetage externe (2 ; 202).

6. Vis intraosseuse (1; 101; 201) selon l'une des revendications précédentes, dans laquelle ledit au moins un filetage externe (2 ; 102 ; 202) présente plusieurs canaux dits capillaires (8 ; 108 ; 208) qui traversent ledit au moins un filetage externe (2; 102; 202) de façon à déboucher dans le logement (6 ; 106 ; 206).

7. Vis intraosseuse (1; 101; 201) selon l'une des revendications précédentes, dans laquelle des entretoises (10; 210), de préférence la majorité des entretoises, présentent des canaux capillaires traversants de façon à déboucher dans le logement logement (6 ; 106 ; 206).

8. Vis intraosseuse (1) selon la revendication 6 ou 7, dans laquelle des canaux capillaires (8) et/ou des orifices traversants (12) ont des formes convergentes depuis une surface externe du filetage externe (2) vers le logement (6), et dans laquelle des canaux capillaires (8) et/ou des orifices traversants (12) ont des formes divergentes depuis une surface externe du filetage externe (2) vers le logement (6).

9. Vis intraosseuse (1; 101; 201) selon l'une des revendications précédentes, dans laquelle ledit au moins un filetage externe (2 ; 102 ; 202) et les moyens de liaison sont respectivement composés de fibres agglomérées (F).

10. Vis intraosseuse (1 ; 101 ; 201) selon la revendication 9, dans laquelle les fibres agglomérées (F) comprennent des fibres composées de polymère d'acide lactique (PLA), et le cas échéant des fibres biorésorbables qui sont de préférence composées de matériaux sélectionnés dans le groupe constitué du collagène, de l'hydroxyapatite et de caprolactone.

11. Vis intraosseuse (201) selon l'une des revendications précédentes, comprenant en outre une tête de vis (201.1) agencée de sorte que la vis intraosseuse (201) peut être serrée dans un alésage après enlèvement préalable d'une portion d'os globalement cylindrique.

12. Vis intraosseuse selon l'une des revendications précédentes, comprenant en outre un filetage dit interne qui s'étend sur les bords du logement.

13. Vis intraosseuse (1; 101; 201) selon l'une des revendications précédentes, dans laquelle chaque entretoise (10 ; 110 ; 210) s'étend entre deux filets consécutifs (4 ; 104 ; 204).

14. Vis intraosseuse (1; 101; 201) selon l'une des revendications précédentes, dans laquelle les entretoises (10 ; 110 ; 210) sont configurées de sorte que la superficie cumulée des orifices traversants (12 ; 112; 212) représente au moins 50%, de préférence au moins 70%, de la surface délimitée entre par les filets (4 ; 104 ; 204).

## Patentansprüche

1. Intraossale Schraube (1; 101; 201), die bestimmt ist, an einem Knochen ein Knochenfragment für eine Osteosynthese oder ein Transplantat für eine Bandplastik zu befestigen, wobei die intraossale Schraube (1; 101; 201) umfasst:
- mindestens ein Außengewinde (2; 102; 202), das von einer Vielzahl von Gängen (4; 104; 204) gebildet ist und das sich gemäß einer Außenspirale um eine Längsachse (X2) erstreckt;
- eine Aufnahme (6), die sich innerhalb des Außengewindes (2; 102; 202) und allgemein parallel zu der Längsachse (X2) erstreckt; und
- Verbindungsmittel, die derart ausgebildet sind, dass die Gänge (4; 104; 204) miteinander verbunden sind;
wobei die Verbindungsmittel Streben (10; 110; 210) umfassen, wobei jede Strebe (10; 110; 210) vorzugsweise mindestens zwei aufeinanderfolgende Gänge (4; 104; 204) verbindet, wobei die Streben (10; 110; 210) derart ausgebildet sind, dass sie eine Vielzahl von Durchgangsöffnungen (12; 112; 212) festlegen, die in die Aufnahme (6) ausmünden,
wobei die Aufnahme (6) ein hohles zylindrisches Volumen begrenzt, das in mindestens ein Ende des Außengewindes (2; 102) ausmündet und angepasst ist, um nach dem Schrauben der intraossalen Schraube (1; 101; 201) in den Knochen einen zylindrischen Knochenabschnitt aufzunehmen,
wobei die intraossale Schraube **dadurch gekennzeichnet ist, dass** das hohle zylindrische Volumen einen Durchmesser (D6) hat, der etwa dem Innendurchmesser (D2) des Endabschnitts des Außengewindes entspricht.

2. Intraossale Schraube (1; 101; 201) nach Anspruch 1, wobei die Querabmessungen der Aufnahme (6; 106; 206) 90 % größer als die minimale Abmessung des Innenteils des Außengewindes (2; 102; 202) sind.

3. Intraossale Schraube (1; 101) nach einem der vorangehenden Ansprüche, wobei das hohle zylindrische Volumen an den zwei Enden des Außengewindes (2; 102) derart ausmündet, dass das Außengewinde (2; 102) nach dem Schrauben der intraossalen Schraube (1; 101) in den Knochen einen Knochenabschnitt aufnehmen kann, der mit dem Knochen fest verbunden bleibt.

4. Intraossale Schraube (1; 101; 201) nach einem der vorangehenden Ansprüche, wobei sich die Streben (10; 110; 210) mindestens paarweise kreuzen.

5. Intraossale Schraube (1; 201) nach Anspruch 4, wobei die Streben (10; 210) umfassen:
- umgekehrte Streben (10.1; 210.1), die sich allgemein gemäß den umgekehrten Spiralen erstrecken, die zu der äußeren Spirale des mindestens einen Außengewindes (2; 202) umgekehrt ausgerichtet sind; und
- direkte Streben (10.2; 210.2), die sich allgemein gemäß den direkten Spiralen erstrecken, die wie die äußere Spirale des mindestens einen Außengewindes (2; 202) ausgerichtet sind.

6. Intraossale Schraube (1; 101; 201) nach einem der vorangehenden Ansprüche, wobei das mindestens eine Außengewinde (2; 102; 202) mehrere Kapillarkanäle (8; 108; 208) aufweist, die das mindestens eine Außengewinde (2; 102; 202) derart durchqueren, dass sie in die Aufnahme (6; 106; 206) ausmünden.

7. Intraossale Schraube (1; 101; 201) nach einem der vorangehenden Ansprüche, wobei Streben (10; 210), vorzugsweise die Mehrheit der Streben, Kapillarkanäle aufweisen, die derart durchquerend sind, dass sie in die Aufnahme (6; 106; 206) ausmünden.

8. Intraossale Schraube (1) nach Anspruch 6 oder 7, wobei Kapillarkanäle (8) und/oder Durchgangsöffnungen (12) ab einer äußeren Oberfläche des Außengewindes (2) in Richtung der Aufnahme (6) konvergierende Formen haben und wobei Kapillarkanäle (8) und/oder Durchgangsöffnungen (12) ab einer äußeren Oberfläche des Außengewindes (2) in Richtung der Aufnahme (6) divergierende Formen haben.

9. Intraossale Schraube (1; 101; 201) nach einem der vorangehenden Ansprüche, wobei das mindestens eine Außengewinde (2; 102; 202) und die Verbindungsmittel jeweils aus Pressfasern (F) zusammengesetzt sind.

10. Intraossale Schraube (1; 101; 201) nach Anspruch 9, wobei die Pressfasern (F) Fasern, die aus Polymilchsäure (PLA) zusammengesetzt sind, und gegebenenfalls biologisch resorbierbare Fasern, die vorzugsweise aus Materialien zusammengesetzt sind, die aus der Gruppe ausgewählt sind, die von dem Kollagen, von dem Hydroxyapatit und von dem Caprolacton gebildet sind, umfassen.

11. Intraossale Schraube (201) nach einem der vorangehenden Ansprüche, umfassend ferner einen Schraubenkopf (201.1), der derart ausgebildet ist, dass die intraossale Schraube (201) nach vorheriger Entfernung eines allgemein zylindrischen Knochenabschnitts in einer Bohrung spannbar ist.

12. Intraossale Schraube nach einem der vorangehenden Ansprüche, umfassend ferner ein Innengewinde, das sich auf den Rändern der Aufnahme erstreckt.

13. Intraossale Schraube (1; 101; 201) nach einem der vorangehenden Ansprüche, wobei sich jede Strebe (10; 110; 210) zwischen zwei aufeinanderfolgenden Gängen (4; 104; 204) erstreckt.

14. Intraossale Schraube (1; 101; 201) nach einem der vorangehenden Ansprüche, wobei die Streben (10; 110; 210) derart konfiguriert sind, dass die kumulierte Oberfläche der Durchgangsöffnungen (12; 112; 212) mindestens 50 %, vorzugsweise mindestens 70 %, der zwischen den Gängen (4; 104; 204) begrenzten Oberfläche darstellt.

## Claims

1. An intraosseous screw (1; 101; 201), intended to attach to a bone a bone fragment for an osteosynthesis or a transplant for a ligament surgery, the intraosseous screw (1; 101; 201) comprising:
- at least one external thread (2; 102; 202) which is formed by a plurality of nets (4; 104; 204) and which extends along a helix called outer helix about a longitudinal axis (X2);
- a housing (6) which extends inside the external thread (2; 102; 202) and generally parallel to the longitudinal axis (X2); and
- connecting means arranged to connect the nets (4; 104; 204) together;
the connecting means comprising spacers (10; 110; 210), each spacer (10; 110; 210) preferably connecting at least two consecutive nets (4; 104; 204), the spacers (10; 110; 210) being arranged to define a plurality of through holes (12; 112; 212) which open into the housing (6),
the housing (6) delimiting a hollow cylindrical volume which opens into at least one end of the external thread (2; 102) and being shaped to house, after screwing of the intraosseous screw (1; 101; 201) in the bone, a portion of a cylindrical bone, the intraosseous screw being **characterized in that** the hollow cylindrical volume has a diameter (D6) substantially equal to the internal diameter (D2) of the end portion of the external thread.

2. The intraosseous screw (1; 101; 201) according to claim 1, wherein the transverse dimensions of the housing (6; 106; 206) are greater than 90% of the minimum dimension of the internal part of the thread external (2; 102; 202).

3. The intraosseous screw (1; 101) according to any of the preceding claims, wherein the hollow cylindrical volume opens into both ends of the external thread (2; 102) so that, after screwing of the intraosseous screw (1; 101) in the bone, the external thread (2; 102) can house a bone portion remaining secured to the bone.

4. The intraosseous screw (1; 101; 201) according to any of the preceding claims, wherein the spacers (10; 110; 210) intersect at least two by two.

5. The intraosseous screw (1; 201) according to claim 4, wherein the spacers (10; 210) comprise:
- spacers called reverse spacers (10.1; 210.1) which generally extend along helices called reverse helices which are oriented reversely to the outer helix of said at least one external thread (2; 202); and
- spacers called direct spacers (10.2; 210.2) which extend generally along helices called direct helices that are oriented as the outer helix of said at least one external thread (2; 202).

6. The intraosseous screw (1; 101; 201) according to any of preceding claims, wherein said at least one external thread (2; 102; 202) has several channels called capillary channels (8; 108; 208) which pass through said at least one external thread (2; 102; 202) so as to open into the housing (6; 106; 206).

7. The intraosseous screw (1; 101; 201) according to any of the preceding claims, wherein spacers (10; 210), preferably the majority of the spacers, have through capillary channels so as to open into the housing (6; 106; 206).

8. The intraosseous screw (1) according to claim 6 or 7, wherein capillary channels (8) and/or through holes (12) have shapes converging from an outer surface of the external thread (2) to the housing (6), and wherein capillary channels (8) and/or through holes (12) have shapes diverging from an outer surface of the external thread (2) to the housing (6).

9. The intraosseous screw (1; 101; 201) according to any of the preceding claims, wherein said at least one external thread (2; 102; 202) and the connecting means are respectively composed of agglomerated fibers (F).

10. The intraosseous screw (1; 101; 201) according to claim 9, wherein the agglomerated fibers (F) comprise fibers composed of lactic acid polymer (PLA), and where appropriate bio-resorbable fibers which are preferably composed of materials selected from the group consisting of collagen, hydroxyapatite and caprolactone.

11. The intraosseous screw (201) according to any of the preceding claims, further comprising a screw head (201.1) arranged so that the intraosseous screw (201) can be tightened in a bore after prior removal of a generally cylindrical bone portion.

12. The intraosseous screw according to any of the preceding claims, further comprising a thread called internal thread which extends over the edges of the housing.

13. The intraosseous screw (1; 101; 201) according to any of the preceding claims, wherein each spacer (10; 110; 210) extends between two consecutive nets (4; 104; 204).

14. The intraosseous screw (1; 101; 201) according to any of the preceding claims, wherein the spacers (10; 110; 210) are configured such that the cumulative surface area of the through holes (12; 112; 212) represents at least 50%, preferably at least 70%, of the delimited surface between the nets (4; 104; 204).
